Europäisches Patentamt

European Patent Office

Office européen des brevets

(19)

(11) Veröffentlichungsnummer : **0 186 238**
**B1**

(12) **EUROPÄISCHE PATENTSCHRIFT**

(45) Veröffentlichungstag der Patentschrift :
07.03.90

(21) Anmeldenummer : 85202037.9

(22) Anmeldetag : 10.12.85

(51) Int. Cl.⁵ : **G 01 N 24/08**

(54) **Verfahren zur Erzeugung eines Bewegungssignals und Kernspintomograph für ein solches Verfahren.**

(30) Priorität : 21.12.84 DE 3446717
21.03.85 DE 3510195

(43) Veröffentlichungstag der Anmeldung :
02.07.86 Patentblatt 86/27

(45) Bekanntmachung des Hinweises auf die Patenterteilung : 07.03.90 Patentblatt 90/10

(84) Benannte Vertragsstaaten :
CH DE FR GB LI NL

(56) Entgegenhaltungen :
EP--A-- 0 117 725
EP--A-- 0 119 802
DE--A-- 2 604 301
US--A-- 3 768 003

(73) Patentinhaber : **Philips Patentverwaltung GmbH**
**Wendenstrasse 35 Postfach 10 51 49**
**D-2000 Hamburg 1 (DE)**
**DE**
**N.V. Philips' Gloeilampenfabrieken**
**Groenewoudseweg 1**
**NL-5621 BA Eindhoven (NL)**
**CH FR GB LI NL**

(72) Erfinder : **Buikman, Dirk, Dr.**
**Bodelschwinghstrasse 20**
**D-2000 Hamburg 63 (DE)**
Erfinder : **Helzel, Thomas**
**Oldesloerstrasse 107**
**D-2000 Hamburg 61 (DE)**
Erfinder : **Röschmann, Peter**
**Steenbalken 13**
**D-2000 Hamburg 63 (DE)**

(74) Vertreter : **Hartmann, Heinrich, Dipl.-Ing. et al**
**Philips Patentverwaltung GmbH Wendenstrasse 35**
**Postfach 10 51 49**
**D-2000 Hamburg 1 (DE)**

## Beschreibung

Die Erfindung betrifft ein Verfahren zum Erzeugen eines von der Bewegung eines in einem Kernspintomographen zu untersuchenden Körpers abhängigen Bewegungssignals. Weiterhin bezieht sich die Erfindung auf einen Kernspintomographen zur Durchführung eines solchen Verfahrens, mit einer ersten Hochfrequenz-Spulenanordnung, die zur Erzeugung eines hochfrequenten Magnetfeldes mit der Lamorfrequenz bzw. zum Empfang von Kernresonanzsignalen dient.

Ein solcher Kernspintomograph ist bekannt. Die Erzeugung des hochfrequenten Magnetfeldes bzw. der Empfang des Kernresonanzsignals erfolgt dabei im allgemeinen mit derselben Hochfrequenz-Spulenanordnung. Der Begriff « Hochfrequenz-Spulenanordnung » ist dabei weit zu interpretieren. Es sind darunter auch Resonatoren zu verstehen, wie sie beispielsweise in der deutschen Offenlegungsschrift DE-A-33 47 597 beschrieben sind. Dadurch wird ein hochfrequentes Magnetfeld erzeugt, dessen Frequenz im Bereich der sogenannten Larmorfrequenz liegt ; die Larmorfrequenz ist der Feldstärke des in dem Kernspintomographen erzeugten statischen Magnetfeldes proportional und beträgt für Wasserstoff rund 42,5 MHz/T. Das hochfrequente Magnetfeld hat in einem zu untersuchenden Körper eine Anregung der Kernspins zur Folge, wodurch nach dem Verschwinden dieses Feldes quasi als Echo in der Hochfrequenz-Spulenanordnung das Kernresonanzsignal induziert wird, das zwecks Bestimmung der Kernspinverteilung und/oder der Relaxationszeiten T1, T2 in dem untersuchten Körper von einem Rechner weiterverarbeitet werden kann.

Die Untersuchung eines Körpers mit einem derartigen Kernspintomographen umfaßt eine Vielzahl von Zyklen, in denen jeweils das hochfrequente Magnetfeld erzeugt und die Kernresonanzsignale empfangen werden. Bevor nach dem Empfang eines Kernresonanzsignals erneut ein hochfrequentes Magnetfeld erzeugt wird, verstreicht ein relativ langer Zeitraum (in der Größenordnung von mehreren 100 ms), so daß die gesamte Untersuchung relativ lange dauert — in der Regel eine Minute oder mehr. Es ist verständlich, daß bei derart langen Zeiträumen Bewegungen des untersuchten Patienten, insbesondere Atem- und Schluckbewegungen unvermeidlich sind. Diese Bewegungen können die Untersuchungsergebnisse verfälschen. Um dies zu vermeiden, ist bei bekannten Kernspintomographen ein Bewegungsdetektor vorgesehen, der Patientenbewegungen detektiert. Ein solcher Bewegungsdetektor, der thermisch, pneumatisch, mechanisch, elektrisch oder optisch wirken kann, muß am Patienten befestigt werden und das von ihm erzeugte Bewegungssignal muß über einen separaten Kanal übertragen werden. Es ist auch bekannt, die Amplitude des NMR-Signals als Bewegungssignal zu verwenden (EP-A-117 725). Mit Hilfe des auf diese Weise erzeugten Bewegungssignals können die Bewegungsartefakte durch die Patientenbewegungen weitgehend unterbunden werden.

Eine Möglichkeit hierzu besteht darin, jeden der zuvor erwähnten Zyklen erst dann zu starten, wenn sich aus dem Bewegungssignal ergibt, daß sich der Patientenkörper wieder in einer definierten Position befindet (sogenanntes Triggering). Eine andere Möglichkeit besteht darin, die Zyklen unabhängig von den Bewegungssignalen des Bewegungsdetektors ablaufen zu lassen — vorzugsweise in konstanten Zeitabständen — und diejenigen Kernresonanzsignale bei der Rekonstruktion nicht zu berücksichtigen, die während einer Bewegung des Körpers aufgetreten sind (sogenanntes Gating). Dabei müssen die nicht berücksichtigten Zyklen jeweils identisch wiederholt werden. - Auch eine Kombination beider Verfahren, Gating und Triggering, ist möglich.

Aufgabe der vorliegenden Erfindung ist es, ein Verfahren anzugeben, mit dem sich Bewegungen einfach detektieren lassen bzw. einen Kernspintomographen zur Durchführung dieses Verfahrens.

Zur Lösung dieser Aufgabe ist bei einem Verfahren der eingangs genannten Art vorgesehen, daß während der Untersuchung die Impedanz einer Hochfrequenz-Spulenanordnung, deren Feld auf den zu untersuchenden Körper einwirkt und die auf eine vorgegebene Resonanzfrequenz abgestimmt ist, im Bereich der Resonanzfrequenz gemessen wird und daß aus dem dabei gewonnenen Meßsignal das Bewegungssignal abgeleitet wird.

Die Erfindung nutzt die Tatsache aus, daß sich bei allen Bewegungen des im Feld der Hochfrequenz-Spulenanordnung befindlichen Patienten z. B. bei Atem-, Schluck-, Herz- und Peristaltik-Bewegungen, die Güte und die Streukapazität der Hochfrequenz-Spulenanordnung ändern. Somit ändert sich auch die Impedanz doeser Spulenanordnung nach Betrag und Phase. Diese Impedanz wird bei der Erfindung während der Untersuchung — zumindest abschnittsweise — gemessen und aus dem dabei erzeugten Meßsignal kann — z. B. durch Gleichrichtung — das Bewegungssignal abgeleitet werden, wobei jedem Wert der Impedanz eine bestimmte Bewegungsphase zugeordnet ist.

Zur Durchführung dieses Verfahrens gibt es zwei Möglichkeiten. Die erste Möglichkeit geht von einem Kernspintomographen aus mit einer Impedanz-Meßeinheit zur Messung der Impedanz der zur Erzeugung eines hochfrequenten Magnetfeldes bzw. zum Empfangen von Kernresonanzsignalen dienenden Hochfrequenz-Spulenanordnung und sie ist dadurch gekennzeichnet, daß die Impedanz-Meßeinheit während der Untersuchung des Körpers aktiviert ist und daß das dabei erzeugte Meßsignal als Bewegungssignal dient.

Es sei an dieser Stelle erwähnt, daß es notwendig ist, die Impedanz der Hochfrequenz-Spulenanordnung eines Kernspintomographen zu messen. Durch das Einbringen des Patienten in den Wirkungsbereich der Hochfrequenz-Spulenanordnung wird deren Güte bzw. Anpassung und deren Resonanzfrequenz nämlich beeinflußt, und deshalb ist die Impedanz-Meßeinheit vorgesehen, die auf Stellglieder zur automatischen Anpassung bzw. Nachstimmung der Hochfrequenz-Spulenanordnung einwirkt. Nach Beginn der eigentlichen Untersuchung ist die Impedanz-Meßeinheit nicht mehr aktiviert; sie kann dann also zur Erzeugung des Bewegungssignals benutzt werden.

Eine zweite Lösung sieht vor, daß der Kernspintomograph zur Erzeugung des Bewegungssignals eine zweite Hochfrequenz-Spulenanordnung aufweist, deren Impedanz von einer Impedanz-Meßeinheit gemessen wird.

Die erste Lösung ist einfacher, weil nur eine Hochfrequenz-Spulenanordnung benötigt wird und weil diese dabei eine Doppelfunktion hat, indem sie einerseits zur Erzeugung eines hochfrequenten Magnetfeldes bzw. zum Empfang von Kernresonanzsignalen dient und andererseits zur Erzeugung des Bewegungssignals. Dafür kann bei der zweiten Lösung die zweite Spulenanordnung jedoch so angeordnet und ausgebildet sein, daß sie wesentlich stärker auf Bewegungen des Körpers anspricht. Außerdem kann hiermit während der gesamten Untersuchung fortlaufend gemessen werden — insbesondere auch bei einer Frequenz, die wesentlich von der Larmorfrequenz abweicht, so daß die eigentliche Untersuchung durch die Erfassung der Bewegungssignale nicht negativ beeinflußt werden kann.

Die Erfindung wird nachstehend anhand der Zeichnungen näher erläutert. Es zeigen :

Fig. 1 einen Längsschnitt durch einen Kernspintomographen in schematischer Darstellung,

Fig. 2 und 3 verschiedene Varianten für einen Kernspintomographen, bei dem die zur Anregung von Kernspins bzw. zum Empfang von Kernresonanzsignalen dienende Hochfrequenz-Spulenanordnung gleichzeitig zur Erzeugung des Bewegungssignals dient,

Fig. 4 eine bei der Ausführungsform nach Fig. 3 verwendbare Schaltung zur Impedanzmessung,

Fig. 5 ein den Betrieb der Hochfrequenz-Spulenaordnung erläuterndes Zeitdiagramm,

Fig. 6 einen Querschnitt durch einen Kernspintomographen mit einer gesonderten Hochfrequenz-Spulenanordnung zur Erfassung des Bewegungssignals,

Fig. 7 eine erste Schaltung zur Impedanzmessung bei einem derartigen Kernspintomographen.

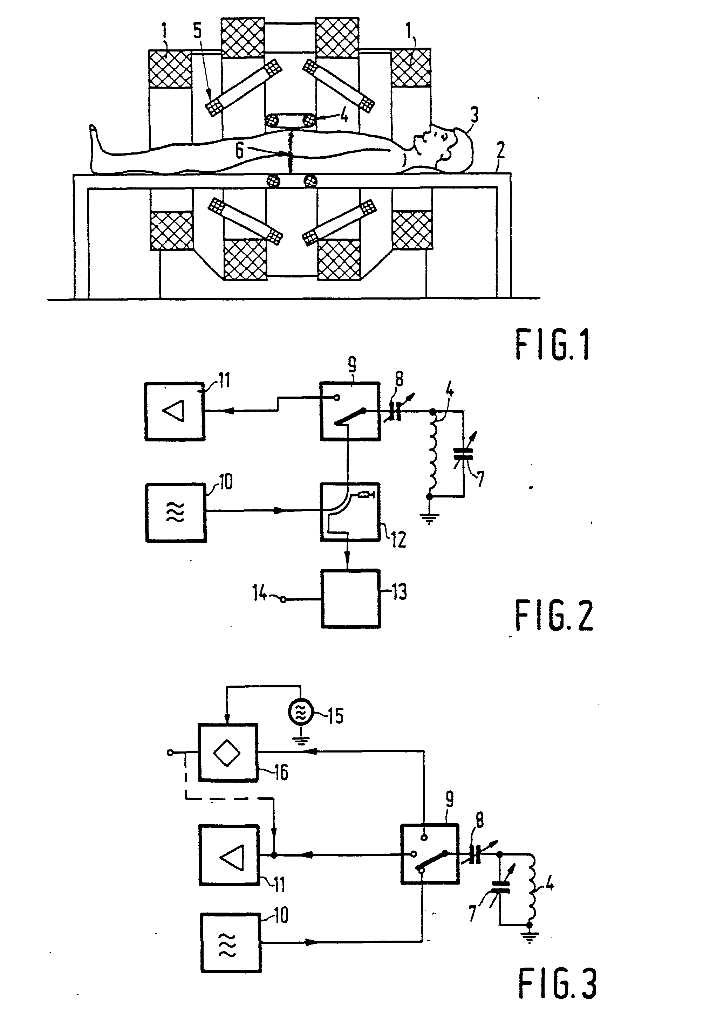

Gemäß Fig. 1 umfaßt ein Kernspintomograph einen aus vier Spulen 1 bestehenden Elektromagneten, der ein starkes statisches, homogenes und in Richtung der gemeinsamen horizontalen Spulenachse verlaufendes Magnetfeld erzeugt. Ein auf einer Tischplatte 2 im Innern des Elektromagneten gelagerter Patient 3 wird von einer Hochfrequenzspule 4 umschlossen, die ein senkrecht zu dem von dem Elektromagneten erzeugten Hauptmagnetfeld gerichtetes hochfrequentes Magnetfeld impulsweise erzeugt. Die Frequenz des hochfrequenten Magnetfeldes ist dabei der Flußdichte des Hauptmagnetfeldes, die je nach Ausführung des Elektromagneten zwischen 0,1 T und 4 T betragen kann, proportional ; die Proportionalitätskonstante entspricht dem gyromagnetischen Verhältnis (ca. 42,5 MHz/T). Innerhalb des von der Hochfrequenzspule umschlossenen Volumens können daher durch Erzeugung des hochfrequenten Magnetfeldes Kernspinresonanzen angeregt werden.

Der Kernspintomograph umfaßt außerdem vier Gradientenspulen 5, die ein in Richtung des Hauptmagnetfeldes verlaufendes und sich in dieser Richtung linear änderndes Magnetfeld erzeugen, sowie weitere Gradientenspulen, die ein ebenfalls in Richtung des Hauptmagnetfeldes verlaufendes Magnetfeld erzeugen, das sich jedoch in zwei dazu senkrechten Richtungen ändert. Durch Erregung dieser Gradientenspulen wird die Phase des im Anschluß an die Erzeugung des hochfrequenten Magnetfeldes in der Hochfrequenzspulenanordnung 4 induzierten Signales in Abhängigkeit von der Kernspinverteilung in dem untersuchten Körperbereich beeinflußt, so daß es grundsätzlich möglich ist, mit einem derartigen Kernspintomographen die Kernspinverteilung in einem zwei- oder dreidimensionalen Bereich eines Körpers zu bestimmen.

In Fig. 2 ist das Blockschaltbild einer ersten Ausführungsform der Erfindung dargestellt. Die Hochfrequenzspulenanordnung 4 ist dabei durch einen Kondensator 7 mit einstellbarer Kapazität, insbesondere einem Drehkondensator, zu einem Parallel-Resonanz-Kreis ergänzt. Der eine Anschluß dieses Parallel-Resonanz-Kreises ist mit Masse verbunden und der andere Anschluß über einen weiteren Drehkondensator 8 mit einem Umschalter 9. In der gezeichneten Stellung des Umschalters 9 wird dem Netzwerk 4, 7, 8 die elektrische Leistung eines leistungsstarken Hochfrequenzsenders 10 zugeführt, dessen Trägerfrequenz der Kernspinresonanzfrequenz entspricht. In der nicht gezeichneten Stellung des Umschalters 9 ist der Vorverstärker 11 eines im übrigen nicht näher dargestellten Empfängers mit dem aus den Drehkondensatoren 7 und 8 und der Hochfrequenzspule 4 bestehenden Netzwerk verbunden, und er kann dann die in der Hochfrequenzspule 4 durch die Kernspinresonanz induzierten Signale empfangen.

Bei der Kernspinresonanzfrequenz sind die Eingangsimpedanz des Vorverstärkers 11, die Ausgangsimpedanz des Hochfrequenzsenders 10 und die Impedanz des Netzwerkes 4, 7, 8 gleich groß und betragen z. B. 50 Ohm. Dieser Zustand der Anpassung wird nach dem Einbringen des Patienten 3 und vor Beginn der eigentlichen Untersuchung durch Abgleich der Kondensatoren 7 und 8 eingestellt, und zwar vorzugsweise automatisch mit Hilfe einer nicht näher dargestellten Impedanzmeßeinrichtung. Dieser Anpassungszustand ändert sich jedoch, wenn der Patient sich innerhalb der Spule bewegt bzw. je nach Lage der Spule auch bei Atem-, Schluck-, Herz- und Peristaltik-Bewegungen, weil dadurch die Güte und die Streukapazität der Spule 4 beeinflußt werden. Der Momentanwert der Impedanz des Netzwerkes 4, 7, 8 ist daher ein Maß für die Bewegungsphase, in der sich der untersuchte Körper jeweils befindet, und deshalb können daraus Bewegungssignale abgeleitet werden.

Zu diesem Zweck ist zwischen den Leistungssender 10 und den Umschalter 9 ein Reflektometer 12 geschaltet, dessen Ausgangssignal in einem Reflexionsmeßempfänger 13 aufbereitet wird, an dessen Ausgang 14 das Bewegungssignal zur Verfügung steht. So lange der Hochfrequenzsender 10 und das Netzwerk 4, 7, 8 einander angepaßt sind, ist das Ausgangssignal des Reflektometers 12 praktisch Null. Bei einer Fehlanpassung wird jedoch ein Teil der zugeführten Hochfrequenzleistung reflektiert, wobei sich am Ausgang des Reflektometers ein hochfrequentes Signal ergibt, dessen Amplitude vom Reflektionsfaktor bzw. von der Fehlanpassung abhängt. Dieses hochfrequente Signal wird im Empfänger 13 gleichgerichtet und ggf. verstärkt und erscheint danach am Ausgang 14. Dieses Signal kann dann mit vorgebbaren Schwellenwerten verglichen werden, die bestimmten Impedanzen und damit bestimmten Bewegungsphasen entsprechen und zur Auslösung von Steuervorgängen herangezogen werden, wenn es sich innerhalb der Schwellenwerte befindet.

In Fig. 5 ist der typische zeitliche Verlauf einer Untersuchung mit einem derartigen Kernspintomographen dargestellt. Während eines Zeitraums Ta wird ein sogenannter 90°-Impuls erzeugt, d. h. der Hochfrequenzsender 10 ist so lange über den Umschalter 9 mit der Hochfrequenzspule 4 verbunden, daß die Kernmagnetisierung in dem untersuchten Körper gerade um 90° aus der Richtung des Hauptmagnetfeldes gekippt wird. Anschließend folgen in der Regel ein oder mehrere sogenannte 180°-Impulse Tb, nach denen jeweils das in der Spule induzierte Signal empfangen wird (Zeitraum Tc), wobei der Umschalter die in der Zeichnung nicht dargestellte Stellung einnimmt. Das ganze dauert typischerweise ca. 100 ms und wiederholt sich periodisch nach einer im Vergleich zu dieser Zeit langen Zeit von typischerweise 600 ms. Bei jeder Wiederholung werden die Felder der Gradientenspule in definierter Weise geändert.

Bei der Anordnung nach Fig. 2 ist eine Impedanzmessung nur möglich, während der Zeiten, während welcher der Spule 4 die elektrische Leistung des Hochfrequenzsenders 10 zugeführt wird — gemäß Fig. 5 also während der Zeiten Ta und Tb. Geht man davon aus, daß während der verhältnismäßig dicht aufeinanderfolgenden Zeiträume Ta...Tc sich der Körper des Patienten in annähernd derselben Phase befindet, dann ist mit Hilfe des gewonnenen Bewegungssignals ein sog. Gating möglich. Dabei wird das in der Spule 4 während des Zeitraums Tc induzierte Kernresonanzsignal ausgewertet, wenn sich das Bewegungssignal in einem bestimmten Amplitudenbereich befindet — wenn also der Patientenkörper sich in einer definierten Lage befindet. Ist dies nicht der Fall, wird das induzierte Signal nicht ausgewertet und die Messung muß wiederholt werden (mit den gleichen Gradientenfeldern).

Wenn mit der Ausführungsform nach Fig. 2 auch ein sog. Triggerring durgeführt werden soll, ist es erforderlich, die Impedanz auch während des relativ langen Zeitraums zwischen dem Empfang des letzten Impulses Tc einer Messung und dem Senden des ersten Impulses Ta einer darauf folgenden Messung zu messen. Deshalb muß der Spule 4 vom Hochfrequenzsender 10 auch während dieses Zeitraums ein Signal zugeführt werden. Damit durch diese Messungen die während des Zeitraums zwischen Tc und Ta stattfindende Kernspinrelaxation nicht beeinflußt wird, muß dies allerdings mit wesentlich reduzierter elektrischer Leistung und/oder in definierten zeitlichen Abständen während dieses Zeitraums mit einer im Vergleich zu den Zeiten für Ta und Tb wesentlich verkürzten Meßzeiten erfolgen, falls nicht bei einer anderen Frequenz als der Larmorfrequenz gemessen wird. Die Erzeugung der Bewegungssignale ist in diesem Fall aber schwieriger, weil das reflektierte Signal entweder eine wesentlich geringere Amplitude hat bzw. während eines wesentlich kürzeren Zeitraums gebildet werden muß.

Durch die Bewegung des Patienten werden — wie bereits erwähnt — Güte und Induktivität der Spule beeinflußt, was u. a. auch eine Verschiebung der Resonanzkurve des Netzwerkes 7, 8, 9 zu höheren oder zu niedrigeren Frequenzen zur Folge hat. Dann existiert jedoch keine eindeutige Beziehung mehr zwischen der Impedanz und der jeweiligen Bewegungsphase. Dies läßt sich vermeiden, wenn das Netzwerk 4, 7, 8 gegenüber der Meßfrequenz — in der Regel der Kernspinresonanzfrequenz — geringfügig verstimmt wird. Die Verstimmung soll dabei klein gegenüber der 3 dB-Bandbreite des Netzwerkes sein, jedoch so groß, daß in jeder möglichen Bewegungsphase die Resonanzfrequenz des Netzwerkes 4, 7, 8 stets entweder unterhalb der Meßfrequenz oder oberhalb der Meßfrequenz bleibt. Ein geeigneter Wert für diese Verstimmung sind 20 kHz bei einer 3 dB-Bandbreite von 300 kHz und einer Kernspinresonanzfrequenz von rund 85 MHz.

Fig. 3 zeigt eine weitere Ausführungsform der Erfindung, wobei gleichartige Teile mit denselben Bezugszeichen versehen sind. Der Umschalter 9 ist dabei direkt mit dem Hochfrequenzsender 10 verbunden, und er besitzt im Gegensatz zu demjenigen nach Fig. 2 drei Schaltstellungen. In der einen Schaltstellung wird die elektrische Leistung des Hochfrequenzsenders 10 dem Netzwerk 4, 7, 8 zugeführt. In einer zweiten Schaltstellung ist der Vorverstärker 11 mit diesem Netzwerk verbunden und in einer dritten Schaltstellung ist seine Impedanz-Meßeinheit an dieses Netzwerk angeschlossen. Die Impedanz-Meßeinheit besteht aus einem zusätzlichen Hochfrequenzgenerator 15 und einer Impedanz-Meßbrücke 16, die vom Hochfrequenzgenerator 15 gespeist wird.

Die Impedanz-Meßbrücke 16, die z. B. nach Art einer Wheatstone'schen Brücke aufgebaut sein kann, ist so ausgelegt, daß sie abgeglichen ist, wenn die Impedanz des Netzwerkes 4, 7, 8 bei der Frequenz des Hochfrequenzgenerators 15 einer bestimmten, für die Anpassung erforderlichen Sollimpedanz, z. B. 50 Ohm entspricht. Der zusätzliche Hochfrequenzgenerator 15 kann wesentlich leistungsschwächer sein als der Hochfrequenzsender 10 und weist diesem gegenüber vorzugsweise eine Frequenzabweichung auf, die klein ist im Vergleich zur 3 dB-Bandbreite des Netzwerkes 4, 7, 8, die jedoch so groß ist, daß die Kernspins in dem zu untersuchenden Bereich des Körpers dadurch nicht angeregt werden. Dadurch wird die eigentliche Untersuchung von der Impedanzmessung nicht beeinflußt. Auch hierbei ist es wiederum von Vorteil, wenn das Netzwerk 4, 7, 8 eine von der Meßfrequenz des zusätzlichen Hochfrequenzgenerators abweichende Resonanzfrequenz hat.

Mit einer derartigen Anordnung ist eine die Impedanzmessung jeweils zwischen den Zeiträumen Ta und Tb, Tb und Tc, Tc und Ta usw. möglich, d. h. immer dann, wenn mit der Hochfrequenzspule 4 kein Magnetfeld erzeugt wird bzw. wenn in ihr kein Kernresonanzsignal induziert wird. Das mit Hilfe der Impedanz-Meßeinheit erzeugte Bewegungssignal kann daher sowohl zum « Gating » als auch zum « Triggerring » benutzt werden.

Das Signal am Ausgang der Impedanz-Meßbrücke bedarf in der Regel noch einer weiteren Verarbeitung (Verstärkung, Gleichrichtung), wozu eine entsprechende Verarbeitungseinheit erforderlich ist. Es ist aber auch möglich, wie durch eine gestrichelte Linie angedeutet, das Ausgangssignal der Impedanz-Meßbrücke 16 durch den Vorverstärker 11 oder einen Teil davon verstärken zu lassen. In diesem Fall ergäbe sich eine Doppelfunktion für den Vorverstärker, die Impedanz-Meßeinheit 15, 16 (Abgleich des Netzwerkes 4, 7, 8 vor Beginn der Untersuchung, Erzeugung des Bewegungssignals während der Untersuchung) und für die Hochfrequenzspule (Bewegungsdetektion, Anregen und Empfangen von Kernresonanzsignalen).

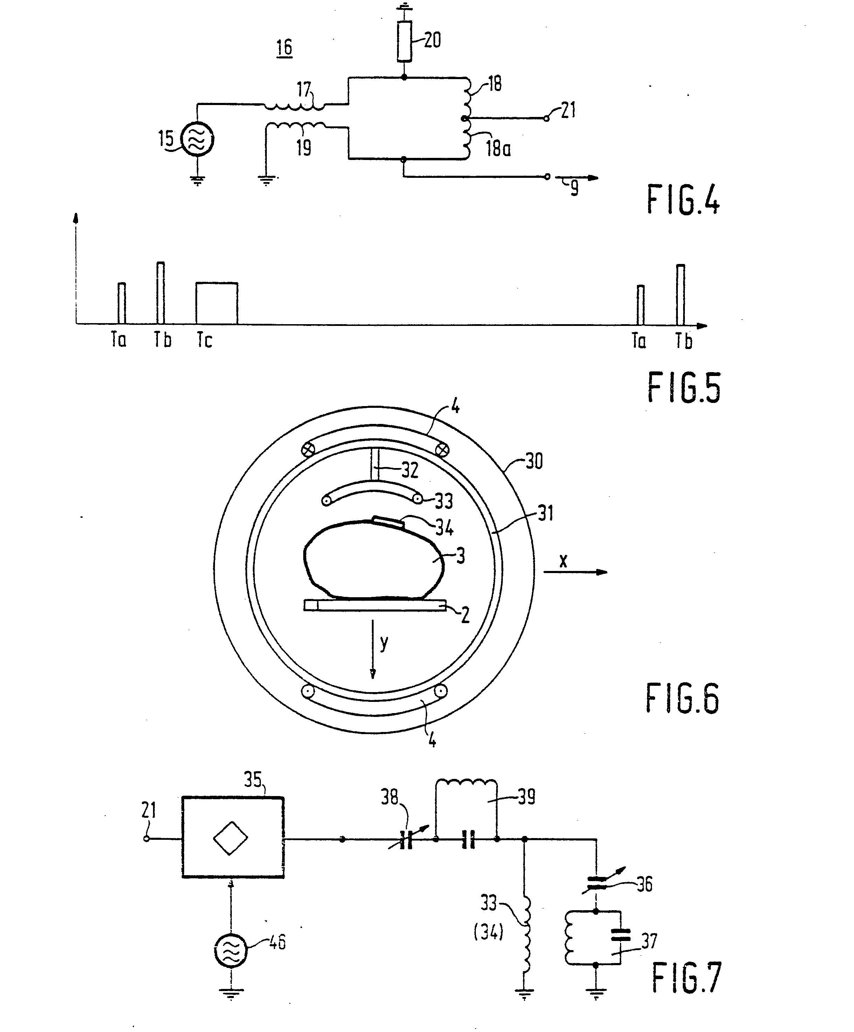

Fig. 4 zeigt eine mögliche Ausführungsform einer Impedanz-Meßeinheit 15, 16 gemäß Fig. 3. Der Hochfrequenzgenerator, dessen einer Anschluß mit Masse verbunden ist, speist dabei eine induktive HF-Brückenschaltung mit vier Induktivitäten 17, 18, 18a und 19, wobei die Induktivitäten 17 und 19 gleich groß sind — ebenso wie die Induktivitäten 18 und 18a. Die beiden Induktivitäten 17 und 19 sind außerdem magnetisch fest miteinander gekoppelt, so daß sich ein Übertrager ergibt. Mit dem Verbindungspunkt der Induktivitäten 17 und 18 ist ein Widerstand 20 verbunden, dessen anderer Anschluß mit Masse verbunden ist und dessen Größe der Größe der Sollimpedanz der HF-Spulenanordnung 10 (50 Ohm) entspricht. Der Verbindungspunkt der Induktivitäten 19 und 18 ist bei der Impedanzmessung über den Umschalter 9 mit dem Netzwerk 4, 7, 8 verbunden, wenn dessen Impedanz bei der Frequenz des Hochfrequenzgenerators 15 gerade den für die Anpassung erforderlichen Wert (50 Ohm) hat, ergeben sich an den Anschlüssen der Induktivität gleich große Spannungen mit entgegengesetzter Phasenlage, so daß an der Mittenanzapfung 21 dieser Induktivität keine Spannung auftritt. Ist die Anpassung nicht gegeben, dann sind die Spannungen an den beiden Enden der Induktivität nicht mehr gleich groß, so daß an der Mittenanzapfung 21 eine deutlich von Null verschiedene Spannung auftritt, die ein Maß für die Fehlanpassung ist.

Vorstehend wurde davon ausgegangen, daß die gleiche Spule, die das hochfrequente Magnetfeld zur Anregung der Kernspins erzeugt, auch die Kernresonanzsignale aufnimmt. Die Erfindung ist jedoch auch dann anwendbar, wenn zum Erregen des Magnetfeldes und zum Empfangen des Kernresonanzsignals je eine gesonderte Spule benutzt werden. Die Ausführungsform nach Fig. 2 ist dann in Verbindung mit der für die Erzeugung des hochfrequenten Magnetfeldes erforderlichen Spule anwendbar, während die Ausführungsform nach Fig. 3 in Verbindung auch mit der dem Aufnehmen des Kernresonanzsignals dienenden Spule angewandt werden kann.

Bisher wurde davon ausgegangen, daß die Impedanzmessung bei einer Frequenz erfolgt, die zumindest in der Nähe der Frequenz des Kernresonanzsignals liegt. Die Messung kann jedoch auch bei einer zweiten, höher liegenden Resonanzfrequenz des Netzwerkes 4, 7 und 8 erfolgen, weil sich dessen Impedanz auch bei der zweiten Resonanzfrequenz stark nach Betrag und/oder Phase ändert. Dies hat den Vorteil, daß die Anregung der Kernspins durch die Impedanzmessung nicht beeinflußt wird.

Bei den bisherigen Ausführungsbeispielen wurde davon ausgegangen, daß die Hochfrequenz-Spulenanordnung einerseits zur Anregung der Kernspins bzw. zum Empfang der Kernresonanzsignale dient und andererseits zur Erfassung des Bewegungssignals. Im folgenden wird eine Ausführungsform beschrieben, bei der zur Erfassung des Bewegungssignals eine gesonderte Hochfrequenz-Spulenanordnung vorgesehen ist.

Fig. 6 zeigt einen Querschnitt durch einen Teil eines Kernspintomographen mit einer solchen gesonderten Hochfrequenzspulenanordnung, wobei im Innern eines kreisförmigen Bereiches 30, der der freien Öffnung des Elektromagneten 1 (Fig. 1), entspricht, die Hochfrequenz-Spulenanordnung 4 in einer für höhere Frequenzen geeigneten Ausführungsform dargestellt ist, wie sie in der deutschen Offenlegungsschrift DE-A-33 47 597 beschrieben ist. Diese zweiteilige Spulenanordnung wird dabei so gespeist, daß in den senkrecht zur Zeichenebene verlaufenden Leitern der oberen Schleife der Strom in der entgegengesetzten Richtung fließt, wie in den entsprechenden Leitern der unteren Schleife. Dadurch wird erreicht, daß das von dieser Spule erzeugte hochfrequente Magnetfeld senkrecht zum statischen Magnetfeld in x-Richtung verläuft.

Die Hochfrequenz-Spulenanordnung 4 ist auf einem hohlzylindrischen Kunststoffkörper 31 angebracht, der auf nicht näher dargestellte Weise starr mit dem Patientenlagerungstisch verbunden ist. An dem Kunststoffkörper 31 ist über einen Träger 32 eine Hochfrequenzspule 33 befestigt, die der Erzeugung von Bewegungssignalen dient. Die Spule kann ggf. auch in y- oder z-Richtung verschiebbar sein. Sie kann aber auch für den Patienten nicht sichtbar mit dem Kunststoffkörper verbunden sein — z. B. auf dessen Außenseite. Sie umfaßt eine oder mehrere Windungen, die so angeordnet sind, daß das dadurch erzeugte Magnetfeld in y-Richtung verläuft. Wenn der Kunststoffkörper 31 mit der Hochfrequenzspulenanordnung 4 sich im Bereich des Kopfes des Patienten befinden würde, könnte die Spule 33 auch so angeordnet sein, daß das von ihr erzeugte Feld in z-Richtung, d. h. in Tischlängsrichtung bzw. senkrecht zur Zeichenebene der Fig. 2 verläuft.

Wichtig ist dabei lediglich, daß das von der Hochfrequenzspule 33 erzeugte Magnetfeld im wesentlichen senkrecht zu dem von der Hochfrequenz-Spulenanordnung 4 erzeugten Magnetfeld verläuft. Dadurch wird nämlich erreicht, daß die Hochfrequenz-Spulenanordnung 4 und die Hochfrequenzspule 33 magnetisch weitgehend voneinander entkoppelt sind, so daß einerseits die Beeinflussung des von der Hochfrequenz-Spulenanordnung 4 erzeugten Magnetfeldes durch das Vorhandensein der Hochfrequenzspule 33 und andererseits die in der Hochfrequenzspule 33 durch die Hochfrequenz-Spulenanordnung 4 induzierten Signale auf ein Minimum herabgesetzt werden.

Mit 34 ist schematisch eine weitere vorzugsweise ebene Spule bezeichnet, die auf dem Patientenkörper 3 so positioniert ist, daß ihr Feld im wesentlichen ebenfalls in y-Richtung verläuft. Diese Spule kann in gleicher Weise aufgebaut sein, wie die in der Kernspintomographie nur zur Aufnahme von Kernresonanzsignalen (und nicht zur Anregung von Kernspins) verwendeten Oberflächenspulen. Im Gegensatz zu der fest mit dem Kunststoffkörper 31 verbundenen Spule 33 kann der Aufbau derartiger Spulen dem jeweiligen Anwendungszweck angepaßt sein, so daß daraus und aus der Tatsache, daß diese Spule sehr dicht an den Bereich herangebracht werden kann, der auf Bewegungen zu überwachen ist, eine wesentlich größere Empfindlichkeit resultiert. Mit derartigen Spulen können daher auch von relativ schwach ausgeprägten Bewegungen, wie sie beispielsweise durch den Herzschlag verursacht werden, auswertbare Bewegungssignale abgeleitet werden. Auf der anderen Seite ist es erforderlich, diese Spulen vor der Untersuchung am Patientenkörper zu fixieren.

In Fig. 7 ist eine Schaltungsanordnung zur Ableitung des Bewegungssignals dargestellt. Die Schaltungsanordnung umfaßt eine Impedanzmeßeinheit 35, beispielsweise eine Impedanzmeßbrücke, an deren einem Zweig ein Hochfrequenzgenerator 46 und an deren anderen Zweig über ein Transformationsnetzwerk die Hochfrequenzspule 33 bzw. 34 angeschlossen ist. Die Frequenz des Hochfrequenzsenders liegt wesentlich höher als die Larmorfrequenz, die sich für Wasserstoff aus der Feldstärke des Magneten ergibt. Beträgt die Feldstärke des Magneten beispielsweise 2 T, dann liegt die Larmorfrequenz bei 85 MHz und die Frequenz des Hochfrequenzsenders sollte dann zwischen 100 MHz und 200 MHz liegen und sie sollte nicht mit einer Harmonischen der Larmorfrequenz zusammenfallen. Wenn mit diesem Kernspintomographen auch die Verteilung anderer Elemente als Wasserstoff erfaßt werden sollen, beispielsweise von Natrium, Phosphor oder Fluor, dann muß die Frequenz darüber hinaus so gewählt werden, daß sie nicht mit Harmonischen der Larmorfrequenzen für Natrium, Phosphor und Fluor zusammenfällt, die bei 2 T etwa bei 68 MHz, 35 MHz und 80 MHz liegen.

Die Transformation der Impedanz der Hochfrequenzspule auf einen vorzugsweise reellen Widerstand von 50 Ohm erfolgt einerseits mittels eines Abstimmkondensators 36, der in Serie mit einem Parallel-Resonanzkreis 37 der Hochfrequenzspule 33 (34) parallelgeschaltet ist, und andererseits über einen Abgleichkondensator 38, über den in Serie mit einem Parallel-Resonanzkreis 39 das eine Ende der Spule 33 (34) mit dem einen Brückenzweig der Impedanzmeßbrücke 35 verbunden ist. Die Parallel-Resonanzkreise 37 und 39 sind auf die Larmorfrequenz abgestimmt und so ausgelegt, daß ihre kapazitive Impedanz bei der Frequenz des Hochfrequenzsenders 36 kleiner ist als die kapazitive Impedanz der Kondensatoren 46 und 38, zumindest aber nicht wesentlich größer, so daß durch Änderung der Kapazität der Kondensatoren 36 und 38 die kapazitive Impedanz im Serien- bzw. im Parallelzweig geändert werden kann.

Die Kondensatoren 36 und 38 werden nach dem Einbringen des Patienten und gegebenenfalls nach dem Fixieren der Hochfrequenzspule 34, jedoch vor Beginn der Untersuchung, so abgeglichen — vorzugsweise automatisch — daß der Eingangswiderstand des durch die Hochfrequenzspule 33 (34) und die Bauelemente 36...39 gebildeten Netzwerkes bei der Frequenz des Hochfrequenzsenders 46 einen bestimmten Wert hat, z. B. 50 Ohm, durch den es an die Impedanzmeßbrücke angepaßt ist. Am Ausgang 21 der Impedanzmeßbrücke 35 erscheint während der gesamten darauf folgenden Untersuchung ein Meßsignal, dessen Amplitude im angepaßten Zustand den Wert Null bzw. ein Minimum aufweist. Bei Bewegungen des Patienten bzw. bei Atem-, Schluck-, Herz- und Peristaltik-Bewegungen ändert sich wie erläutert die Güte und die Streukapazität der Spule 33 bzw. 34, was eine Änderung der Impedanz des mit der Impedanzmeßbrücke 35 verbundenen Netzwerkes zur Folge hat, so daß die Amplitude des Ausgangssignals entsprechend der jeweiligen Bewegungsphase zunimmt. Das Signal am Ausgang 21 kann — vorzugsweise nach Gleichrichtung mit kleiner Zeitkonstante — als Bewegungssignal dienen.

Im allgemeinen läßt sich nicht völlig vermeiden, daß durch das von der Hochfrequenz-Spulenanordnung 4 erzeugte Magnetfeld eine Spannung in der zur Erzeugung von Bewegungssignalen dienenden Hochfrequenzspule 33 und 34 induziert wird; dies gilt insbesondere, wenn — wie bei der Hochfrequenzspule 34 — deren Lage in bezug auf die Hochfrequenz-Spulenanordnung veränderbar ist. Dadurch fließen in der mit der Hochfrequenzspule 33 bzw. 34 verbundenen Schaltung Ströme, wodurch die Hochfrequenz-Spulenanordnung 4 belastet und das von ihr erzeugte Magnetfeld verzerrt wird. Außerdem kann das Signal am Ausgang 21 verfälscht werden, wobei man sich vor Augen halten muß, daß die Leistung, mit der die Hochfrequenz-Spulenanordnung 4 beaufschlagt wird, wesentlich höher ist als die der Hochfrequenzspule 33 bzw. 34 zugeführte elektrische Leistung. Beide Effekte werden durch die Einschaltung der Resonanzkreise 37 und 39 reduziert, die bei der Frequenz der Hochfrequenz-Spulenanordnung 4 eine hohe Impedanz aufweisen.

Durch die Komponenten 36 und 37 wird erreicht, daß die Hochfrequenzspule 33 bzw. 34 bei der Frequenz des Hochfrequenzsenders 46 in Parallelresonanz betrieben wird. Wird die Hochfrequenzspule 33 (34) hingegen durch einen in Serie geschalteten Abstimmkondensator 36 in Serienresonanz betrieben, dann kann der parallelgeschaltete Serienzweig mit dem Parallel-Resonanzkreis 37 entfallen, weil der dann gebildete Serien-Resonanzkreis ohnehin bei weit von seiner Serienresonanzfrequenz entfernten Frequenzen — wie der Frequenz der Hochfrequenz-Spulenanordnung 4 — eine hohe Impedanz aufweist.

Ein Vorteil der Ausführungsform mit einer gesonderten Hochfrequenz-Spulenanordnung zur Erfassung des Bewegungssignals besteht darin, daß der Bewegungszustand des Patienten während der gesamten bzw. nahezu der gesamten Untersuchung fortlaufend erfaßt werden kann. Außerdem können durch das von der Hochfrequenzspule 33 bzw. 34 erzeugte Magnetfeld keine Kernspins angeregt werden, weil die Frequenz des Hochfrequenzsenders wesentlich von der Larmorfrequenz abweicht.

## Patentansprüche

1. Verfahren zum Erzeugen eines von der Bewegung eines in einem Kernspintomographen zu untersuchenden Körpers abhängigen Bewegungssignals, dadurch gekennzeichnet, daß während der Untersuchung die Impedanz einer Hochfrequenz-Spulenanordnung (33; 34), deren Feld auf den zu untersuchenden Körper (3) einwirkt und die auf eine vorgegebene Resonanzfrequenz abgestimmt ist, im Bereich der Resonanzfrequenz gemessen wird und daß aus dem dabei gewonnenen Meßsignal das Bewegungssignal abgeleitet wird.

2. Kernspintomograph zur Durchführung des Verfahrens nach Anspruch 1 mit einer ersten Hochfrequenz-Spulenanordnung (4), die zur Erzeugung eines hochfrequenten Magnetfeldes mit der Lamorfrequenz bzw. zum Empfang von Kernresonanzsignalen dient, dadurch gekennzeichnet, daß zur Erzeugung des Bewegungssignals eine zweite Hochfrequenz-Spulenanordnung (33; 34) vorgesehen ist, deren Impedanz von einer Impedanzmeßeinheit (35; 41) gemessen wird.

3. Kernspintomograph nach Anspruch 2, dadurch gekennzeichnet, daß die zweite Hochfrequenz-Spulenanordnung (33; 34) auf eine Resonanzfrequenz abgestimmt ist, die wesentlich von der Protonen-Resonanzfrequenz abweicht und vorzugsweise wesentlich höher liegt. als diese.

4. Kernspintomograph nach Anspruch 2 oder 3, dadurch gekennzeichnet, daß die zweite Hochfrequenz-Spulenanordnung (33; 34) so angeordnet ist, daß das von ihr erzeugte Magnetfeld wenigstens annähernd senkrecht zum Magnetfeld der ersten Hochfrequenz-Spulenanordnung (4) verläuft.

5. Kernspintomograph nach einem der Ansprüche 2 bis 4, dadurch gekennzeichnet, daß die zweite Hochfrequenz-Spulenanordnung (33; 34) derart in eine Filteranordnung (36...39) aufgenommen ist, daß dem Feld der ersten Hochfrequenz-Spulenanordnung durch die zweite Hochfrequenz-Spulenanordnung möglichst wenig Energie entzogen wird.

6. Kernspintomograph nach einem der Ansprüche 2 bis 5, dadurch gekennzeichnet, daß eine Umschaltanordnung (43) vorgesehen ist, die während der Erzeugung des Magnetfeldes durch die erste Hochfrequenz-Spulenanordnung das Bewegungssignal unterdrückt.

7. Kernspintomograph nach einem der Ansprüche 2 bis 6, dadurch gekennzeichnet, daß die zweite Hochfrequenz-Spulenanordnung (33) mechanisch starr oder allenfalls in einer Ebene verschiebbar mit der ersten Hochfrequenz-Spulenanordnung gekoppelt ist.

8. Kernspintomograph nach einem der Ansprüche 2 bis 6, dadurch gekennzeichnet, daß die zweite Hochfrequenz-Spulenanordnung (34) unabhängig von der ersten Hochfrequenz-Spulenanordnung positionierbar ist.

9. Kernspintomograph zur Durchführung des Verfahrens nach Anspruch 1 mit einer Impedanz-Meßeinheit zur Messung der Impedanz der zur Erzeugung eines hochfrequenten Magnetfeldes bzw. zum Empfang von Kernresonanzsignalen dienenden Hochfrequenz-Spulenanordnung, dadurch gekennzeichnet, daß die Impedanzmeßeinheit (12, 15, 16; 15, 23) während der Untersuchung des Körpers (3) aktiviert ist und daß das dabei erzeugte Meßsignal als Bewegungssignal dient.

10. Kernspintomograph nach Anspruch 9, dadurch gekennzeichnet, daß die Impedanz-Meßeinheit während der Erzeugung des HF-Magnetfeldes durch die Hohfrequenzspulenanordnung (4) aktiviert ist. (Fig. 2)

11. Kernspintomograph nach Anspruch 10, dadurch gekennzeichnet, daß die Impedanz-Meßeinheit (12) als Reflektometer ausgebildet und zwischen die Hochfrequenzspulenanordnung (4) und einen diese speisenden Hochfrequenz-Generator (10) geschaltet ist.

12. Kernspintomograph nach Anspruch 9, dadurch gekennzeichnet, daß die Hochfrequenzspulenanordnung (4) über eine Umschalteranordnung (9) in einer ersten Schaltstellung mit einem Hochfrequenz-Generator (10), in einer zweiten Schaltstellung mit einem Empfänger (11) zum Empfang der Kernresonanzsignale und in einer dritten Schaltstellung mit der Impedanz-Meßeinrichtung (15, 16) verbunden ist, daß die Umschalteranordnung sich während der Untersuchung auch in der dritten Schaltstellung befindet und daß die Impedanz-Meßeinrichtung (15, 16) aktivierbar ist, wenn sie mit der Hochfrequenzspulenanordnung gekoppelt ist.

13. Kernspintomograph nach Anspruch 12 oder einem der Ansprüche 2 bis 8, dadurch gekennzeichnet, daß die Impedanzmessung bei einer von der Kernspinresonanzfrequenz abweichenden Frequenz erfolgt.

14. Kernspintomograph nach Anspruch 9, dadurch gekennzeichnet, daß die Impedanz-Meßeinheit die Impedanz bei einer zweiten Resonanzfrequenz der Hochfrequenzspulenanordnung mißt, die wesentlich höher liegt als die Frequenz der Kernresonanzsignale.

15. Kernspintomograph nach einem der Ansprüche 2 oder 4 bis 14, dadurch gekennzeichnet, daß die Meßfrequenz, bei der die Impedanzmeßeinheit die Impedanz der Hochfrequenzspulenanordnung (4 ; 33, 34) mißt, geringfügig von der Resonanzfrequenz der Hochfrequenzspulenanordnung abweicht.

## Claims

1. A method of generating a movement signal which is dependent on the motion of a body to be examined in a nuclear spin tomograph, characterized in that during the examination the impedance of a high-frequency coil system (33, 34) whose field acts on the body (3) to be examined and which is tuned to a predetermined resonance frequency is measured in the resonance frequency range, the movement signal being derived from the measurement signal thus formed.

2. A nuclear spin tomograph for performing the method claimed in claim 1, including a first high-frequency coil system (4) for generating a high-frequency magnetic field having the Larmor frequency and for receiving spin resonance signals, characterized in that for generating the movement signal there is provided a second high-frequency coil system (33, 34) whose impendance is measured by an impedance measuring unit (35 ; 41).

3. A nuclear spin tomograph as claimed in claim 2, characterized in that the second high-frequency coil system (33 ; 34) is tuned to a resonance frequency which deviates essentially from the proton resonance frequency and which is preferably substantially higher than the latter frequency.

4. A nuclear spin tomograph as claimed in claim 2 or 3, characterized in that the second high-frequency coil system (33 ; 34) is arranged so that the magnetic field generated thereby extends at least substantially perpendicularly to the magnetic field of the first high-frequency coil system (4).

5. A nuclear spin tomograph as claimed in any one of the Claims 2 to 4, characterized in that the second high-frequency coil system (33 ; 34) is included in a filter device (36...39) in such a manner that a small as possible amount of energy is withdrawn from the first high-frequency coil system by the second high-frequency coil system.

6. A nuclear spin tomograph as claimed in any one of the Claims 2 to 5, characterized in that there is provided a switching device (43) which suppresses the movement signal during the generating of the magnetic field by the first high-frequency coil system.

7. A nuclear spin tomograph as claimed in any one of the Claims 2 to 6, characterized in that the second high-frequency coil system (33) is mechanically rigidly coupled to the first high-frequency coil system or is at the most slidable in one plane.

8. A nuclear spin tomograph as claimed in any one of the claims 2 to 6, characterized in that the second high-frequency coil system (34) can be positioned independently from the first high-frequency coil system.

9. A nuclear spin tomograph for performing the method claimed in claim 1, including an impedance measuring unit for measuring the impedance of the high-frequency coil system used for generating a high-frequency magnetic field and for receiving spin resonance signals, characterized in that the impedance measuring unit (12, 15, 16 ; 15, 23) is activated during the examination of the body (3), the measurement signal thus generated being used as the movement signal.

10. A nuclear spin tomograph as claimed in Claim 9, characterized in that the impedance measuring unit is activated during the generating of the high-frequency magnetic field by the high-frequency coil system (4) (Fig. 2).

11. A nuclear spin tomograph as claimed in Claim 10, characterized in that the impedance measuring unit (12) is constructed as a reflectometer connected between the high-frequency coil system (4) and a high-frequency generator (10) which feeds this high-frequency coil system.

12. A nuclear tomograph as claimed in Claim 9, characterized in that the high-frequency coil system (4) is connected, via a switching device (9), to a high-frequency generator (10) in a first switching position, to a receiver (11) for receiving the spin resonance signals in a second switching position, and to the impedance measuring (device (15, 16) in a third switching position, the switching device being in the third switching position also during the examination, the impedance measuring device (15, 16) being activatable when it is coupled to the high-frequency coil system.

13. A nuclear spin tomograph as claimed in Claim 12 or any one of the Claims 2 to 8, characterized in that the impedance measurement is performed at a frequency which deviates from the spin resonance frequency.

14. A nuclear spin tomograph as claimed in claim 9, characterized in that the impedance measuring unit measures the impedance at a second resonance frequency of the high-frequency coil system which is substantially higher than the frequency of the spin resonance signals.

15. A nuclear spin tomograph as claimed in any one of the Claims 2 or 4 to 14, characterized in that the measurement frequency at which the impedance measuring unit measures the impedance of the high-frequency coil system (4 ; 33, 34) deviates slightly from the resonance frequency of the high-frequency coil system.

## Revendications

1. Procédé pour produire un signal de mouvement dépendant du mouvement d'un corps à examiner dans un tomographe à résonance magnétique nucléaire, caractérisé en ce que, pendant l'examen, l'impédance d'un agencement de bobine radiofréquence (33 ; 34), dont le champ agit sur le corps à examiner (3) et qui est accordé sur une fréquence de résonance prédéfinie, est mesurée dans le domaine de la fréquence de résonance et que le signal de mouvement est dérivé du signal de mesure ainsi obtenu.

2. Tomographe à résonance magnétique nucléaire pour l'exécution du procédé suivant la revendication 1, comprenant un premier agencement de bobine radiofréquence (4) qui sert à produire un champ magnétique radiofréquence ayant la fréquence de Larmor ou à recevoir les signaux de résonance magnétique nucléaire, caractérisé en ce que, pour produire le signal de mouvement, il est prévu un deuxième agencement de bobine radiofréquence (33 ; 34) dont l'impédance est mesurée à l'aide d'une unité de mesure d'impédance (35 ; 41).

3. Tomographe à résonance magnétique nucléaire suivant la revendication 2, caractérisé en ce que le second agencement de bobine radiofréquence (33 ; 34) est accordé sur une fréquence de résonance qui diffère notablement de la fréquence de résonance des protons et est, de préférence, sensiblement supérieure à cette dernière.

4. Tomographe à résonance magnétique nucléaire suivant la revendication 2 ou 3, caractérisé en ce que le second agencement de bobine radiofréquence (33 ; 34) est monté de façon que le champ magnétique qu'il produit s'étende au moins à peu près perpendiculairement au champ magnétique du premier agencement de bobine radiofréquence (4).

5. Tomographe à résonance magnétique nucléaire suivant l'une quelconque des revendications 2 à 4, caractérisé en ce que le deuxième agencement de bobine radiofréquence (33 ; 34) est incorporé dans un montage de filtre (36...39), de façon qu'une quantité d'énergie aussi faible que possible soit soustraite au champ du premier agencement de bobine radiofréquence par le deuxième agencement de bobine radiofréquence.

6. Tomographe à résonance magnétique nucléaire suivant l'une quelconque des revendications 2 à 5, caractérisé en ce qu'il est prévu un montage de commutation (43) qui supprime le signal de mouvement pendant la production du champ magnétique par le premier agencement de bobine radiofréquence.

7. Tomographe à résonance magnétique nucléaire suivant l'une quelconque des revendications 2 à 6, caractérisé en ce que le deuxième agencement de bobine radiofréquence (33) est couplé avec le premier agencement de bobine radiofréquence de façon mécaniquement rigide ou, en tout cas, avec translation possible dans un plan.

8. Tomographe à résonance magnétique nucléaire suivant l'une quelconque des revendications 2 à 6, caractérisé en ce que le second agencement de bobine radiofréquence (34) est positionnable indépendamment du premier agencement de bobine radiofréquence.

9. Tomographe à résonance magnétique nucléaire pour l'exécution du procédé suivant la revendication 1, comprenant une unité de mesure d'impédance pour la mesure de l'impédance de l'agencement de bobine radiofréquence servant à produire un champ magnétique radiofréquence ou à recevoir les signaux de résonance magnétique nucléaire, caractérisé en ce que l'unité de mesure d'impédance (12, 15, 16 ; 15, 23) est activée pendant l'examen du corps (3) et que le signal de mesure ainsi obtenu sert de signal de mouvement.

10. Tomographe à résonance magnétique nucléaire suivant la revendication 9, caractérisé en ce que l'unité de mesure d'impédance est activée pendant la production du champ magnétique RF par l'agencement de bobine radiofréquence (4) (Fig. 2).

11. Tomographe à résonance magnétique nucléaire suivant la revendication 10, caractérisé en ce que l'unité de mesure d'impédance (12) est construite comme un réflectomètre et est connectée entre l'agencement de bobine radiofréquence (4) et un générateur de radiofréquence (10) alimentant celui-ci.

12. Tomographe à résonance magnétique nucléaire suivant la revendication 9, caractérisé en ce que l'agencement de bobine radiofréquence (4) est connecté, par l'intermédiaire d'un montage de commutation (9), dans une première position de commutation, à un générateur de radiofréquence (10), dans une seconde position de commutation, à un récepteur (11) pour recevoir les signaux de résonance magnétique nucléaire et dans une troisième position de commutation, à l'unité de mesure d'impédance (15, 16), que le montage de commutation se trouve pendant l'examen aussi dans la troisième position de commutation et que l'unité de mesure d'impédance (15, 16) peut être activée lorsqu'elle est couplée à l'agencement de bobine radiofréquence.

13. Tomographe à résonance magnétique nucléaire suivant la revendication 12 ou l'une quelconque des revendications 2 à 8, caractérisé en ce que la mesure d'impédance a lieu à une fréquence qui diffère de la fréquence de résonance magnétique nucléaire.

14. Tomographe à résonance magnétique nucléaire suivant la revendication 9, caractérisé en ce que l'unité de mesure d'impédance mesure l'impédance à une deuxième fréquence de résonance de l'agencement de bobine radiofréquence qui est sensiblement supérieure à la fréquence des signaux de résonance magnétique nucléaire.

15. Tomographe à résonance magnétique nucléaire suivant l'une quelconque des revendications 2 ou 4 à 14, caractérisé en ce que la fréquence de mesure, à laquelle l'unité de mesure d'impédance mesure l'impédance de l'agencement de bobine radiofréquence (4 ; 33, 34) diffère peu de la fréquence de résonance de l'agencement de bobine radiofréquence.

FIG.1

FIG.2

FIG.3

FIG.4

FIG.5

FIG.6

FIG.7